# EUROPEAN PATENT APPLICATION

(11) **EP 3 217 177 A1**
(43) Date of publication of application: **13.09.2017**
(21) Application number: 16159351.2
(22) Date of filing: 09.03.2016
(51) Int. Cl.: G01N 33/574

(54) **METHODS FOR PROGNOSIS AND TREATMENT OF COLORECTAL CANCER**

(71) Applicant: University of Limerick, Limerick (IE)
(72) Inventor: Dowling, Catriona, Limerick (IE); Kiely, Patrick, Limerick (IE)
(74) Representative: Carr, Anne Marie

(57) **Abstract**

A method for making a prognosis of colorectal cancer in a patient suffering from colorectal cancer is provided wherein the method comprises comparing PKC Beta II levels in a biological sample of colorectal cancer epithelial tissue and in a biological sample of normal distant epithelial tissue from the patient, wherein higher levels of PKC Beta II in the normal distant epithelial tissue sample than in the cancer sample indicate increased disease free survival. Also provided is a method for determining a suitable treatment for a patient suffering from colorectal cancer and a method for treating colorectal cancer comprising administering PKC Beta II or a drug which upregulates expression and/or activity of PKC Beta II to a subject in need thereof.

## Description

### Field of the Invention

The invention relates to methods for determining a prognosis of colorectal cancer in a patient suffering from colorectal cancer. The prognosis may be used to help to determine appropriate treatments for the patient and the invention extends to methods for treating a patient with colorectal cancer.

### Background of the Invention

Colorectal cancer is a highly prevalent form of cancer. Globally, colorectal cancer is the third most common type of cancer making up about 10% of all cases.

The Protein Kinase C (PKC) family is a family of serine/threonine kinases whose 9 members are expressed in many different tissue types and have diverse biological functions. The PKC isozymes are responsible for mediating several biological processes including cell-cycle regulation, cell survival, cell adhesion and regulation of apoptosis. All members of the PKC family share common basic structures - a flexible hinge segment linking a cell membrane targeting N-terminal regulatory moiety to a C-terminal catalytic domain. The regulatory moiety contains two discrete membrane targeting modules and a pseudosubstrate segment that maintain the enzyme in an inactive conformation. The maturation of PKCs into this autoinhibited conformation is dependent on sequential phosphorylation steps at three highly conserved sites termed the activation loop, the turn motif, and the hydrophobic motif. Following these phosphorylation events, the protein is activated by specific secondary messengers that bind to the regulatory domain to mediate the release of the pseudosubstrate segment from the active site. The classical PKCs are targeted by diaclyglycerol (DAG) and Ca2+, the novel PKCs are activated by DAG alone, and the atypical PKCs bind neither DAG nor Ca2+ and instead are regulated by protein:protein interactions. Prolonged activation of PKCs with phorbol esters results in a downregulation of the protein as a result of dephosphorylation and subsequent degradation.

Research into the role of different PKC isoforms in cancer is based primarily on the assumption that increased PKC activation and expression promotes carcinogen induced tumorigenesis. A large body of evidence supports this, for example, overexpression of PKCε is a suggested tumour promoter in stomach, lung, thyroid, colon and breast cancer, increased levels of PKCη are associated with tumour aggressiveness and an increased rate of cell proliferation in non-small-cell lung cancer and overexpression of PKC θ is linked to gastrointestinal stromal tumours. However, immunohistochemical and biochemical studies indicate that altered expression of the PKC isoforms is variable and depends on the cancer type. For example, it has been shown that PKCα can both induce and supress colon cancer cell proliferation. Similarly, PKCζ overexpression in colon cancer cell lines decreases tumour formation in nude mice while loss of PKCζ is also associated with decreased tumorigenicity. The expression of PKCβ in breast, gastric and colon cancer has been subject to much debate and there are studies presenting arguments for both up and down regulation of the isoform in cancer cells and cancer tissue.

Collectively, this research has driven the development of several target therapies against cancer. These targeted therapies are showing promise as potential drugs in the treatment of cardiac diseases and bipolar mania, suggesting that there is merit in targeting PKC. However, despite extensive efforts, cancer therapies directed at the PKC family of serine/threonine kinases have failed in clinical trials. These therapies have been directed at inhibiting PKC and have, in some cases, worsened disease outcome. Meta-analysis conducted on controlled clinical trials of chemotherapy alone versus chemotherapy combined with PKC inhibitors discovered response rates and disease control rates were significantly reduced in the groups that were administrated PKC inhibitors (Zhang L et al., Clinical and Translational Oncology. 2014:1-7.). Recent work examining PKC mutations in human cancers revealed that approximately 60% of PKC mutations actually reduced or abolished PKC activity and none were activating (Antal CE et al., Cell. 2015; 160:489-502). This study also demonstrated that correction of one loss of function PKC Beta II mutation in a colon cancer cell line actually decreased tumour size in mouse xenografts.

As PKCs are differentially expressed across and within different tissues, there is a need to clarify the role played by different members of the PKC family in different types of cancer. This may ultimately help in improved methods of prognosis and treatment of cancer.

### Summary of the Invention

According to a first aspect of the present invention there is provided a method for making a prognosis of colorectal cancer in a patient suffering from colorectal cancer, the method comprising determining PKC Beta II levels in a biological sample of normal distant epithelial tissue from the patient.

Specifically the method for making a prognosis of colorectal cancer in a patient suffering from colorectal cancer according to the first aspect of the invention comprises steps of:
- determining PKC Beta II levels in a biological sample of colorectal cancer epithelial tissue from the patient;
- determining PKC Beta II levels in a biological sample of normal distant epithelial tissue from the patient; and
- comparing determined levels of PKC Beta II in the cancer and normal distant epithelial tissue samples to make the prognosis.

In certain embodiments the step of comparing determined levels of PKC Beta II in the cancer and normal distant epithelial tissue samples comprises calculating a percentage difference between the determined levels.

Typically if the determined levels of PKC Beta II in the normal distant epithelial tissue sample are similar to or lower than the determined levels of PKC Beta II in the cancer sample, the prognosis is poor. In particular, it is expected that disease free survival of the patient will be reduced in patients with low levels of PKC Beta II in their normal distant epithelial tissue when compared to patients with high levels of PKC Beta II in their normal distant epithelial tissue. A poor prognosis typically predicts a disease free survival period of less than ten years. Low levels in a patient's normal distant epithelium tissue can be determined by comparing with levels in the patient's cancer epithelium tissue. Low levels in normal distant epithelium tissue are defined as levels that are lower than, or similar to, levels in cancer epithelium tissue. Levels are considered similar if the percentage difference between the levels is 10% or less. In alternative embodiments, levels are considered similar if the percentage difference is 15% or less, 14% or less, 13% or less, 12% or less, 11% or less, 9% or less, 8% or less, 7% or less, 6% or less, 5% or less, 4% or less, 3% or less, 2% or less or 1% or less. In particular, levels may be considered similar to each other if the percentage difference is 5% or less.

Typically if the determined levels of PKC Beta II in the normal distant epithelial tissue sample are higher than the determined levels of PKC Beta II in the cancer sample and the percentage difference therebetween is more than 10%, the prognosis is good. In alternative embodiments, if the determined levels of PKC Beta II in the normal distant epithelial tissue sample are higher than the determined levels of PKC Beta II in the cancer sample and the percentage difference therebetween is more than 15%, 14%, 13%, 12%, 11%, 9%, 8%, 7%, 6%, 5%, 4%, 3%, 2% or 1%, the prognosis is good. In particular, it is expected that disease free survival of the patient will be increased in patients with high levels of PKC Beta II in their normal distant epithelial tissue when compared to patients with low levels of PKC Beta II in their normal distant epithelial tissue. In particular, a good prognosis typically predicts a disease free survival period of more than ten years. High levels in a patient's normal distant epithelium tissue can be determined by comparing with levels in the patient's cancer epithelium tissue. Levels in normal distant epithelium tissue are defined as high where the levels are higher than in the cancer epithelium tissue and the percentage difference between levels in normal distant epithelium tissue and cancer epithelium tissue is more than 10%. In alternative embodiments, levels are considered high if the levels are higher than in the cancer epithelium tissue and the percentage difference is more than 5%.

A sample comprising normal distant epithelial tissue may be defined as a sample of tissue 5-15cm (+/- 2cm) from the colorectal cancer tissue in the patient.

The colorectal cancer sample may be colon or rectal tissue.

In certain embodiments the method comprises determining PKC Beta II gene expression levels and/or determining PKC Beta II protein levels. Typically this is carried out *in vitro.*

In certain embodiments the method further comprises deciding on a treatment for the patient based on the prognosis. For example, in the event that the prognosis is poor, the method may include deciding on a treatment which is more aggressive. In certain embodiments, the treatment may upregulate expression and/or activity of PKC Beta II. Accordingly in certain embodiments the method includes a step of treating the patient to upregulate expression and/or activity of PKC Beta II.

According to a second aspect of the present invention, there is provided a method for determining a suitable treatment for a patient suffering from colorectal cancer, the method comprising determining PKC Beta II levels in a biological sample of colorectal cancer tissue from the patient.

Specifically the method for determining a suitable treatment for a patient suffering from colorectal cancer according to the second aspect of the invention comprises steps of:
- determining PKC Beta II levels in a biological sample of colorectal cancer tissue from the patient;
- determining PKC Beta II levels in a biological sample of normal tissue from the patient; and
- comparing determined levels of PKC Beta II in the cancer and normal tissue samples to determine a suitable treatment.

In certain embodiments the step of comparing determined levels of PKC Beta II in the cancer and normal tissue samples comprises calculating a percentage difference between the determined levels.

Typically if the determined levels of PKC Beta II in the normal tissue sample are higher than the determined levels of PKC Beta II in the colorectal cancer sample and a percentage difference therebetween is more than 10%, a suitable treatment may comprise upregulating expression and/or activity of PKC Beta II. In alternative embodiments, if the determined levels of PKC Beta II in the normal tissue sample are higher than the determined levels of PKC Beta II in the colorectal cancer sample and the percentage difference is more than 15%, 14%, 13%, 12%, 11%, 9%, 8%, 7%, 6%, 5%, 4%, 3%, 2% or 1%, a suitable treatment may comprise upregulating expression and/or activity of PKC Beta II.

The sample of colorectal cancer tissue may be epithelial or stromal tissue or a mixture thereof. The colorectal cancer sample may be colon or rectal tissue.

The sample of normal tissue may be a sample of normal distant tissue, which may be defined as a sample 5-15cm (+/- 2cm) from the colorectal cancer tissue in the patient. Alternatively the sample of normal tissue may be a sample of normal adjacent tissue, which may be defined as a sample 0-5 cm (+/- 2cm) from the colorectal cancer tissue in the patient. The sample of normal tissue may be epithelial or stromal tissue or a mixture thereof.

In certain embodiments the method comprises determining PKC Beta II gene expression levels and/or determining PKC Beta II protein levels. Typically this is carried out *in vitro.*

According to a third aspect of the present invention, there is provided a method for treating colorectal cancer, the method comprising upregulating expression and/or activity of PKC Beta II to a subject in need thereof. In certain embodiments, the method comprises administering a drug or applying a procedure which upregulates expression and/or activity of PKC Beta II to the subject. In certain embodiments, the method comprises administering PKC Beta II to the subject.

A subject in need thereof may be a subject who is suffering from colorectal cancer. Typically the subject has lower levels of PKC Beta II (gene expression or protein) in the subject's colorectal cancer tissue than in the subject's normal colorectal tissue, as per the second aspect of the present invention. In particular, levels of PKC Beta II in the subject's normal tissue sample are typically higher than levels of PKC Beta II in the subject's colorectal cancer sample and a percentage difference therebetween is typically more than 10%.

Also provided is PKC Beta II or a drug which upregulates expression and/or activity of PKC Beta II for use in treatment of colorectal cancer in a subject in need thereof.

Further provided is use of PKC Beta II or a drug which upregulates expression and/or activity of PKC Beta II in preparation of a medicament for treatment of colorectal cancer in a subject in need thereof.

### Brief Description of the Figures

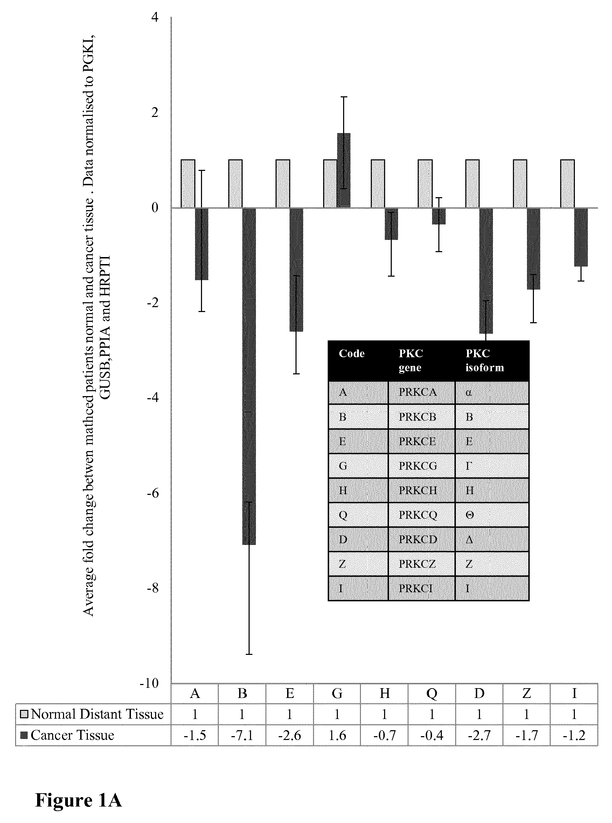

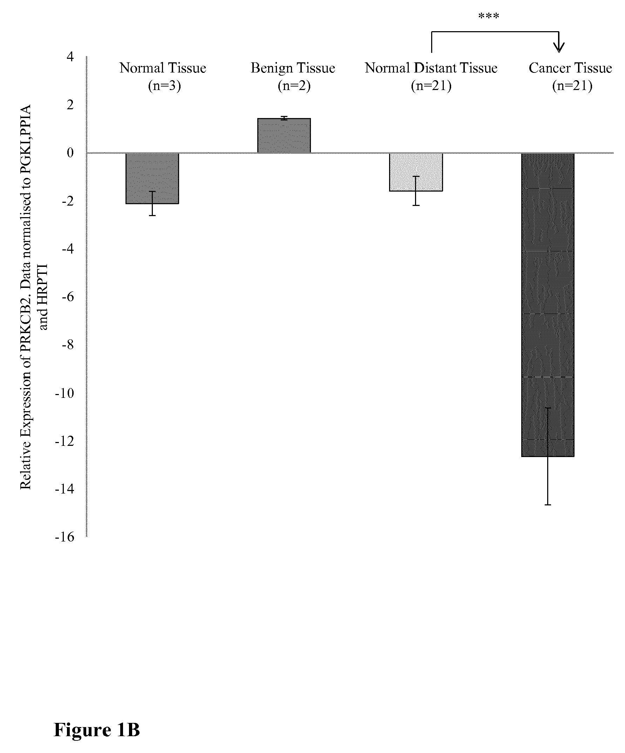

Figure 1. Gene expression of PKC genes in colorectal cancer. Tissue samples measuring approximately 0.5cm in diameter were collected from 21 patients undergoing surgery in University Hospital Limerick. Normal tissue from the 21 patients was also collected approximately 10 cm away from the cancer tissue. RNA was extracted from the tissue, cDNA was synthesised and real time PCR was carried out. All data was normalized using the housekeeping genes PRGK1, GUSB, PPIA and HRPT1. (A) Average fold change of the 9 PKC coding genes in cancer tissue of patients compared to each patients normal tissue (n=21). Each individual patient's fold change was analysed using the REST© software. The table explains the corresponding PKC coding gene and PKC isoform. (B) Gene expression of PRKCB2 in normal tissue (n=3), benign tissue (n=2) and cancer tissue (n=21) relative to normal distant tissue (n=21). Data was analysed using the delta delta ct method (Statistical significance based on the Welch test and Bonferroni test ***p<0.01).

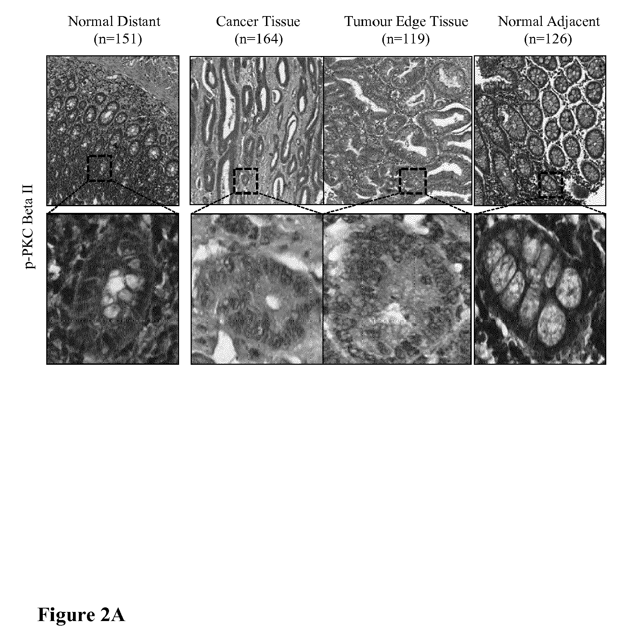

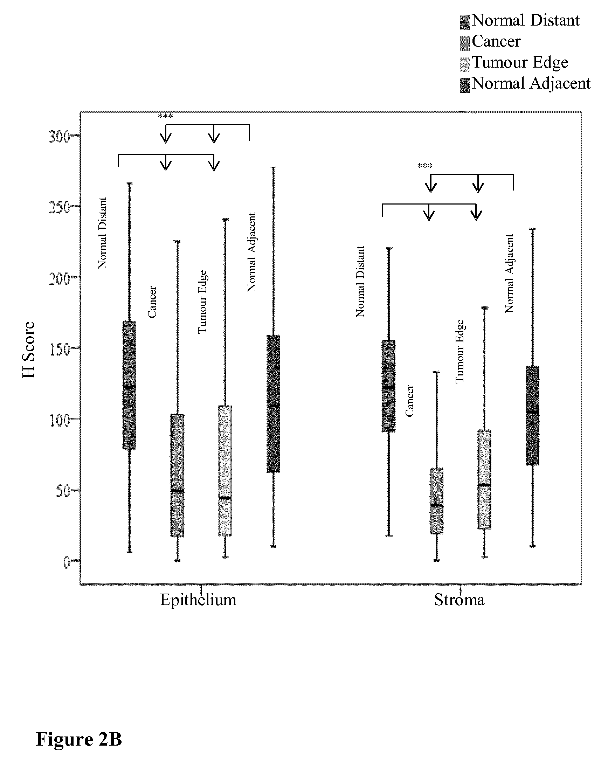

Figure 2. Expression of PKC Beta II in CRC patients. PKC Beta II expression was assessed by immunohistochemistry in normal distant, cancer, tumour edge and normal adjacent tissue samples from CRC patients (n=166). (A) Representative image of PKC Beta II staining for Normal Distant (n=151), Cancer (n=164), Tumour Edge (n=119) and Normal Adjacent (n=126) tissue. (Top panel images = 5X, Bottom panel images = 20X). (B) Box plot representing the difference in PKC Beta II H score between normal distant, cancer, tumour edge and normal adjacent in epithelium (n=151,164,119,126) and stromal tissue (n=152,166,121,126) (Statistical difference based on Welch Test and Bonferroni test, ***p<0.001).

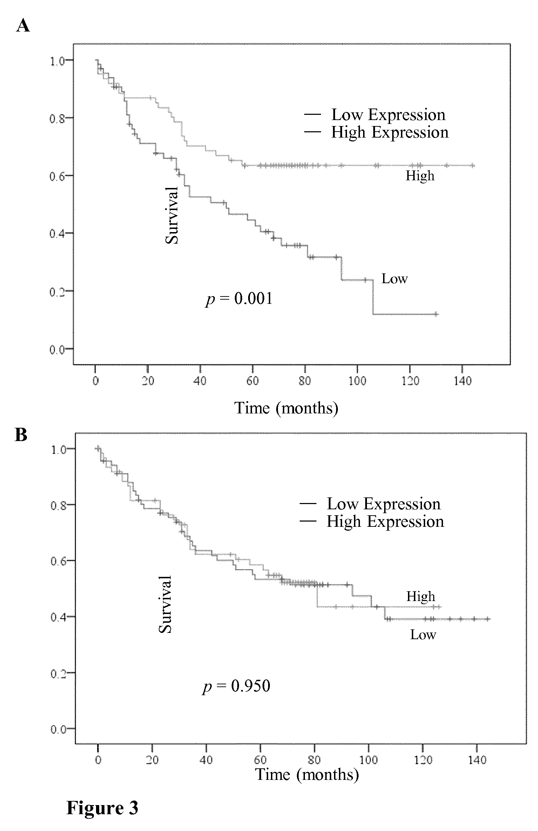

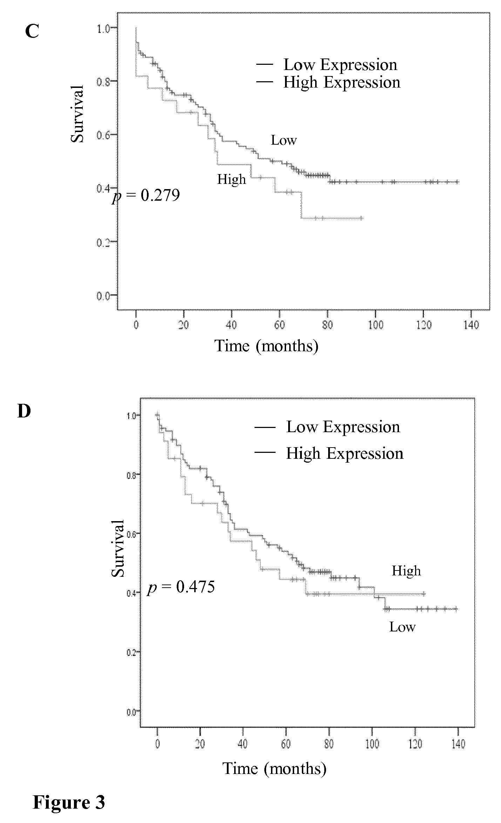

Figure 3. Disease Free Survival of patients with high and low expression of PKC Beta II. High and low expression of PKC Beta II was determined using the median H score value for normal distant tissue and cancer tissue. (A) Effect of high (n=61) and low (n=65) expression of PKC Beta II on disease free survival in the normal distant epithelium tissue of CRC patients. (B) Effect of high (n=75) and low (n=77) expression of PKC Beta II on disease free survival in the normal distant stromal tissue of CRC patients. (C) Effect of high (n=22) and low (n=126) expression of PKC Beta II on disease free survival in the cancer epithelium tissue of CRC patients. (D) Effect of high (n=40) and low (n=126) expression of PKC Beta II on disease free survival in the cancer stromal tissue of CRC patients. P-values represent the Log-rank test.

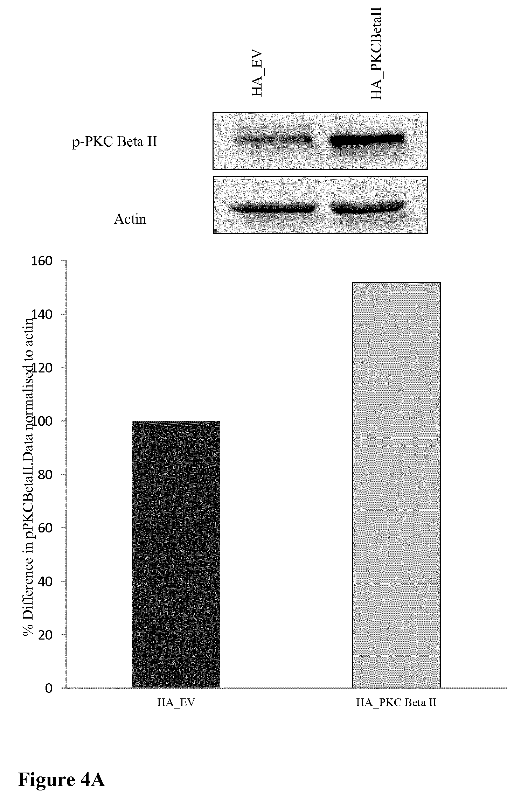

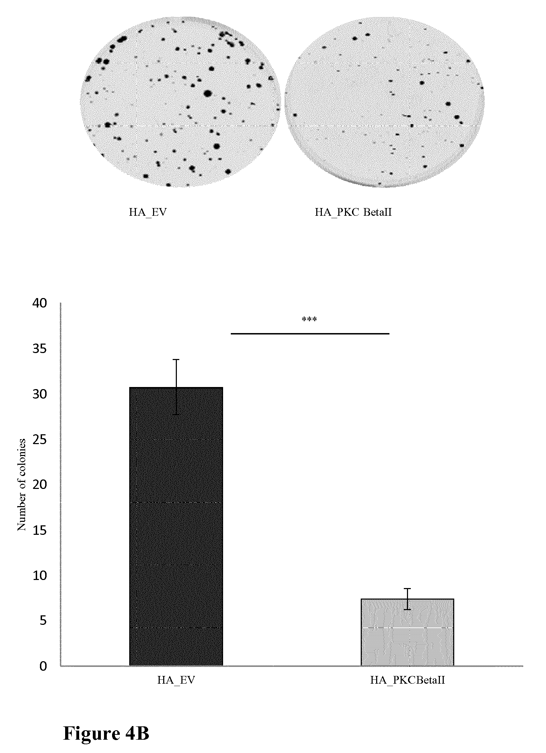

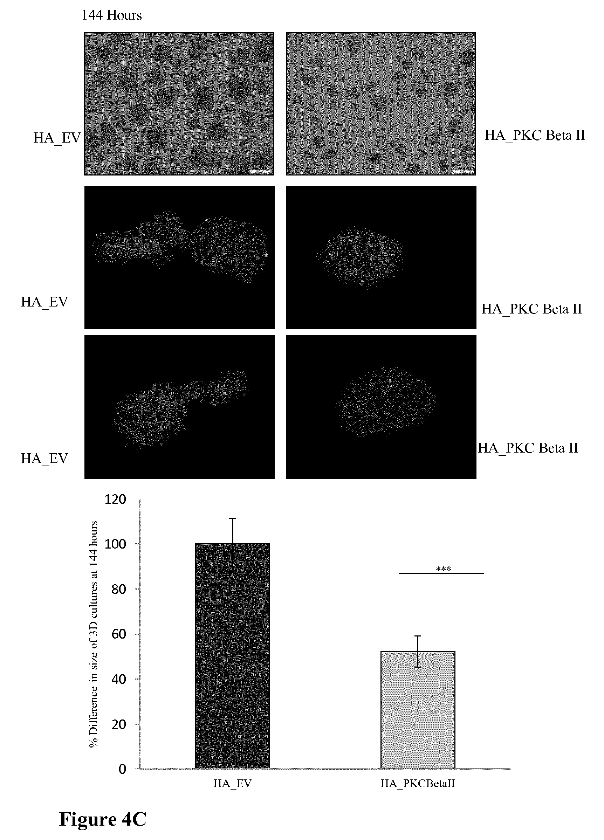

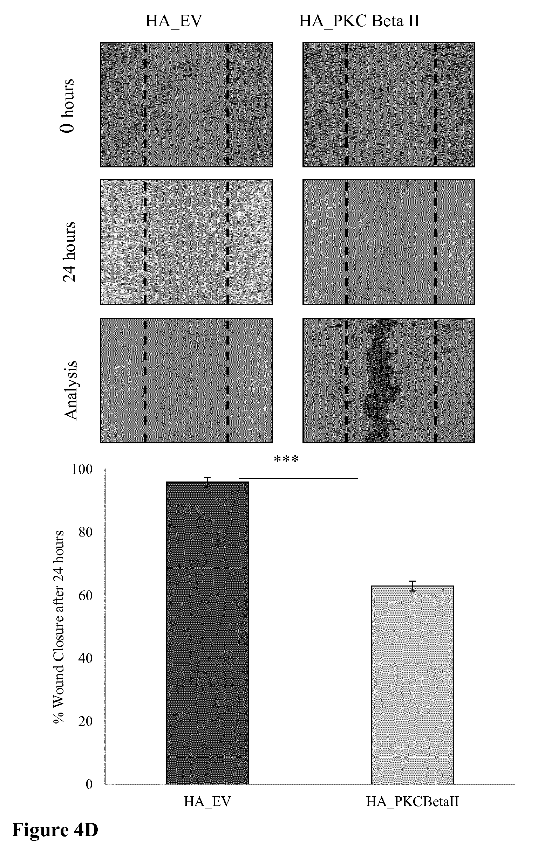

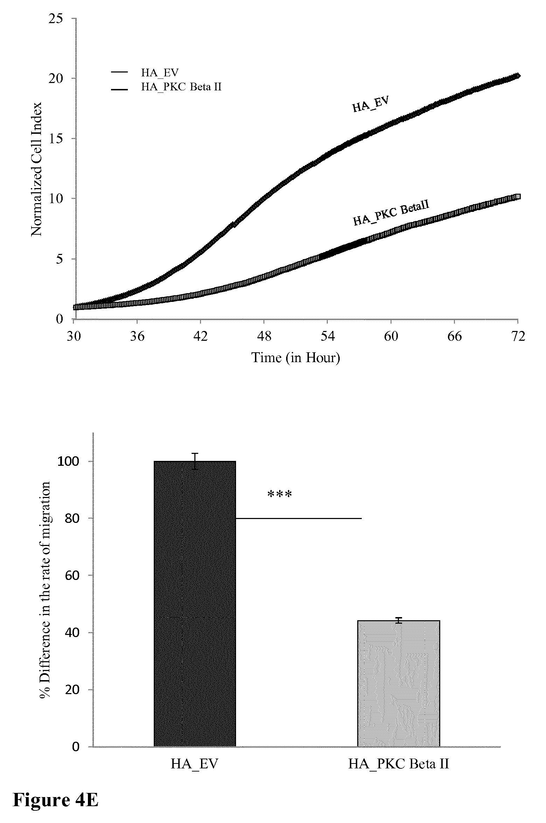

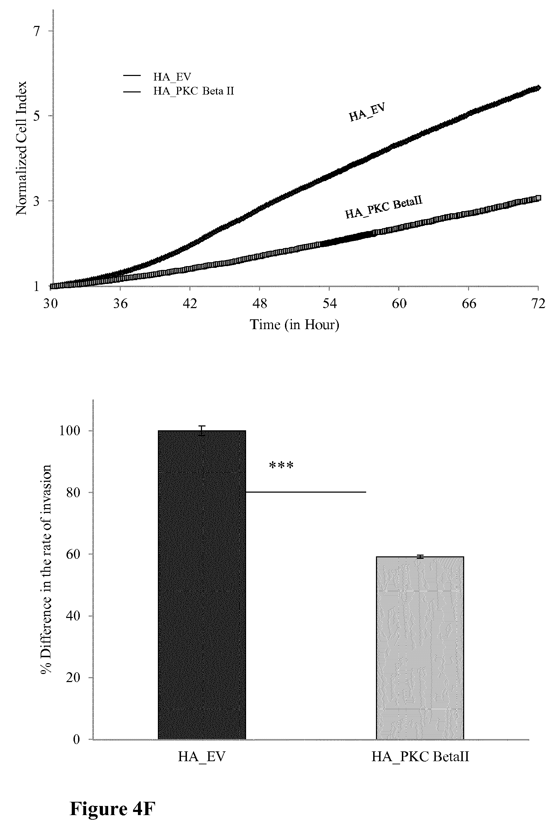

Figure 4. Effect of overexpression of PKC Beta II on colorectal cancer cells. The effect of overexpressing PKC Beta II in colorectal cancer cells was examined using HCT116 cells stably over expressing HA_PKCBeta II. (A) Representative western blot showing the expression of p-PKC Beta II in stable overexpressing HCT116 cells (n=3). (B) Representative images demonstrating the difference in the cells ability to form colonies when overexpressing PKC Beta II (n=3). (C) Images demonstrating the difference in the size of cells growing in a 3D matrix over 144 hours (n=3). Bar graph represents the percentage difference in the size of 3D cultures at 144 hours. Confocal images are cells harvested and stained after 144 hours (n=3). (D) Representative image of cells that were scored with a wound and allowed to migrate over 24 hours. Bar graph represents the percentage wound closer after 24 hours (n=3). (E) Representative graph of cells migrating over a period of 72 hours analysed in real time on the xCELLigence system (n=3). Bar graph represents the percentage difference in cell index. (F) Representative graph of cells invading over a period of 72 hours analysed in real time on the xCELLigence system (n=3). Bar graph represents the percentage difference in cell index.

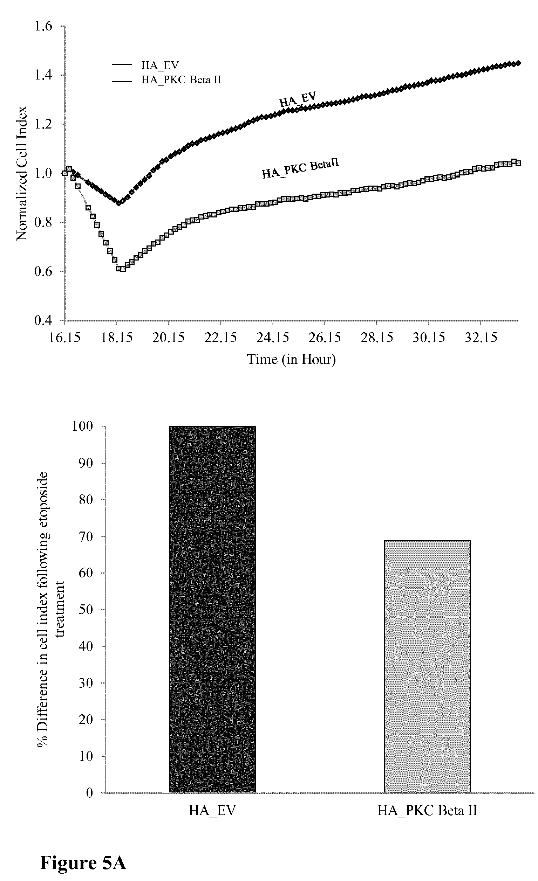

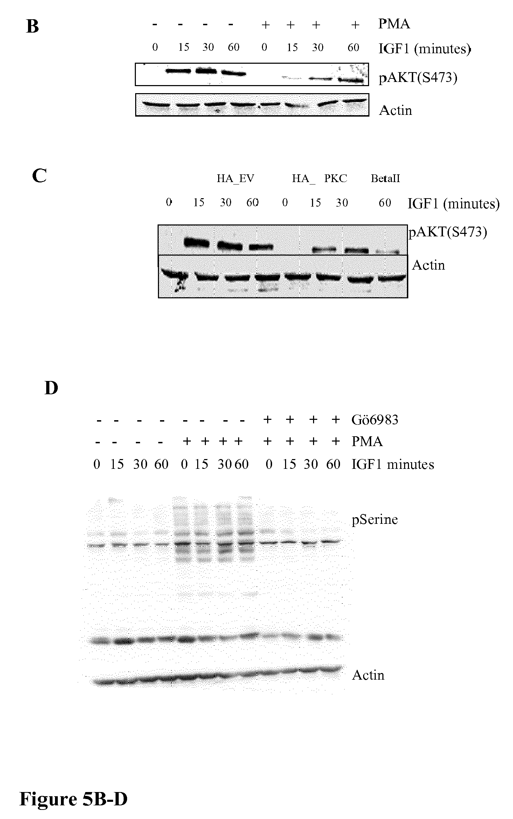

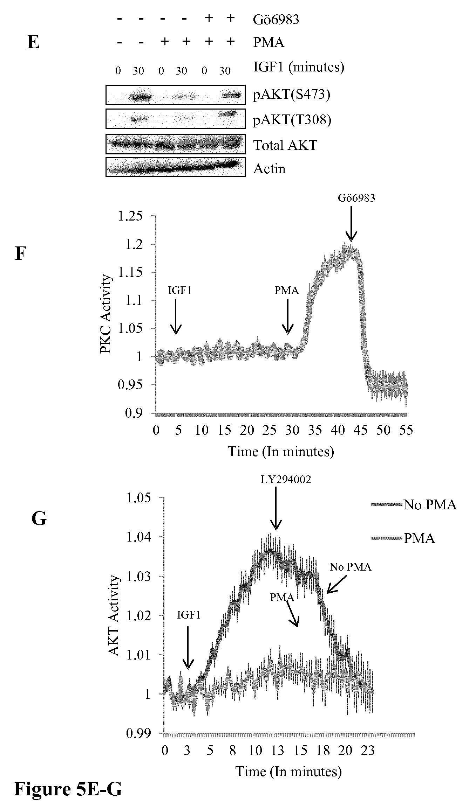

Figure 5. Effect of overexpressing and activating PKC Beta II on protection from cell death and IGF-1 mediated AKT phosphorylation. The effect of overexpressing PKC Beta II on protection from etoposide was examined using HCT116 cells stably overexpressing HA_PKCBeta II. Phorbol 12-myristate 13-acetate (PMA) was utilized to activate PKCs following which IGF-1 mediated AKT phosphorylation was examined. (A) Representative graph of cells proliferating following etoposide treatment at 16hours. Data analysed in real time on the xCELLigence system (n=3). Bar graph represents the percentage difference in cell index. (B) Representative western blot showing the effect of PMA treatment on the IGF-1 mediated expression of pAKT in HCT116 cells (n=3). (C) Representative western blot indicating the difference in IGF-1 mediated phosphorylation of AKT when HCT116 cells are stably overexpressing PKC Beta II (n=3). (D) Representative western blot showing the activation of PKC with the addition of PMA and the inhibition of PKC with the addition of Gö6983 (n=3). (E) Representative western blot showing the effect of a PKC inhibitor on the PMA induced downregulation of pAKT (n=3). (F) Normalised FRET ratio changes (mean +/- SEM) showing PKC activity from HCT116 cells co-expressing CKAR and RFP-tagged PKC Beta II treated with IGF-1, PMA and Gö6983. (G) Normalised FRET ratio changes (mean +/- SEM) showing AKT activity from HCT116 cells expressing plasma membrane targeted BKAR treated with IGF-1 and LY294002.

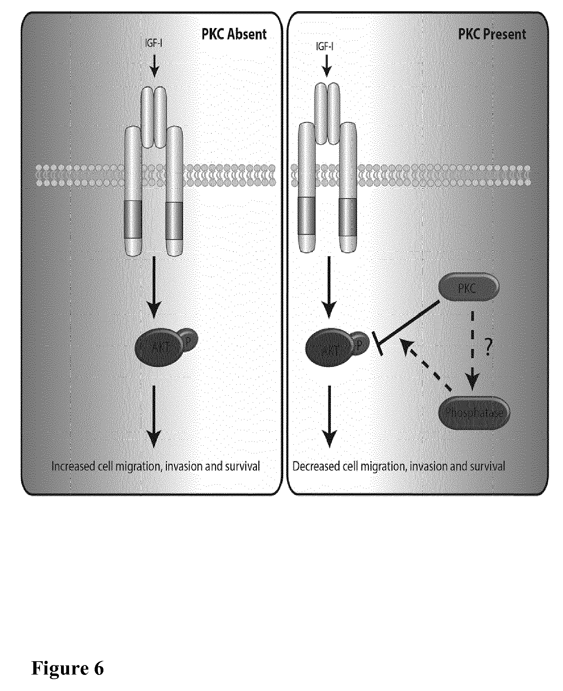

Figure 6. Model demonstrating how the absence or presence of PKC determines the rate of cell migration, invasion and survival.

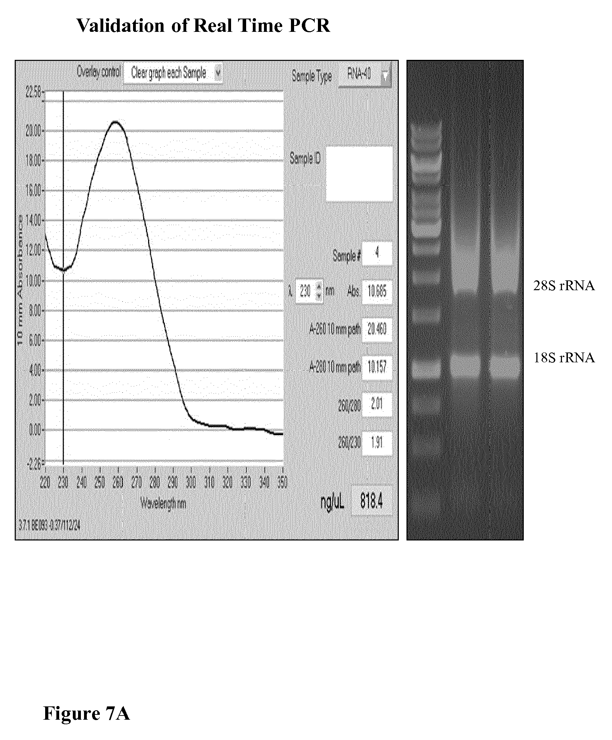

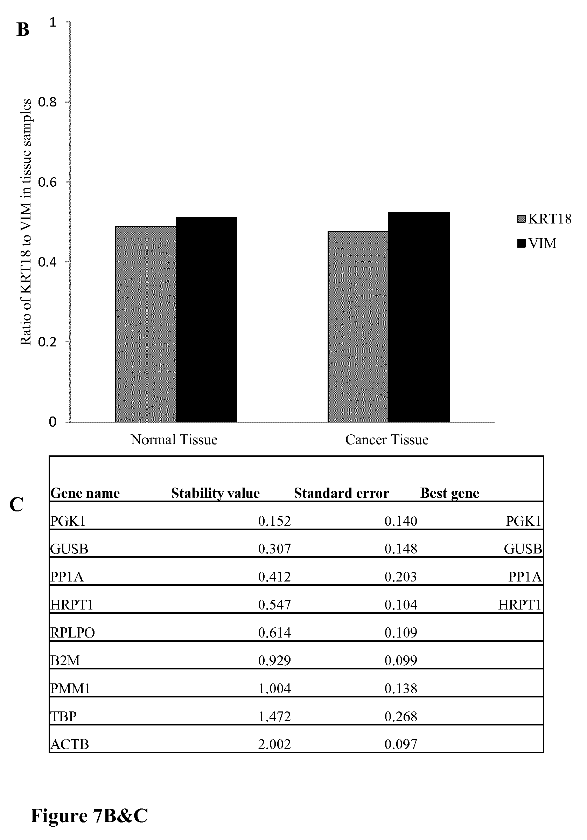

Figure 7. Validation of Real Time PCR. RNA purity and quality was analysed for all samples using the ratio of the absorbance at 260 and 280 nm. The RNA integrity was analysed for all samples by visualization of the 28S:18S ribosomal RNA ratio on a 1% agarose gel. The ratio of stromal tissue to epithelium tissue in normal tissue and cancer tissue samples was analysed using KRT18 (epithelium marker) and VIM (stromal marker) in 12 random tissue samples. To identify the best housekeeping genes to normalize the data a panel of 9 housekeeping genes were tested against tissue samples and analysed in excel using Normfinder. (A) Representative graph from Nanodrop Spectrophotometer displaying the quantity and purity of the RNA. Representative image of agarose gel displaying the 28S:18S ribosomal RNA ratio for a cancer and matched normal sample. (B) The ratio of epithelium to stromal tissue was analysed based on the expression of KRT18 and VIM in the normal and cancer tissue. (C) Table of results displaying the stability value of all 9 genes tested based on normfinder.

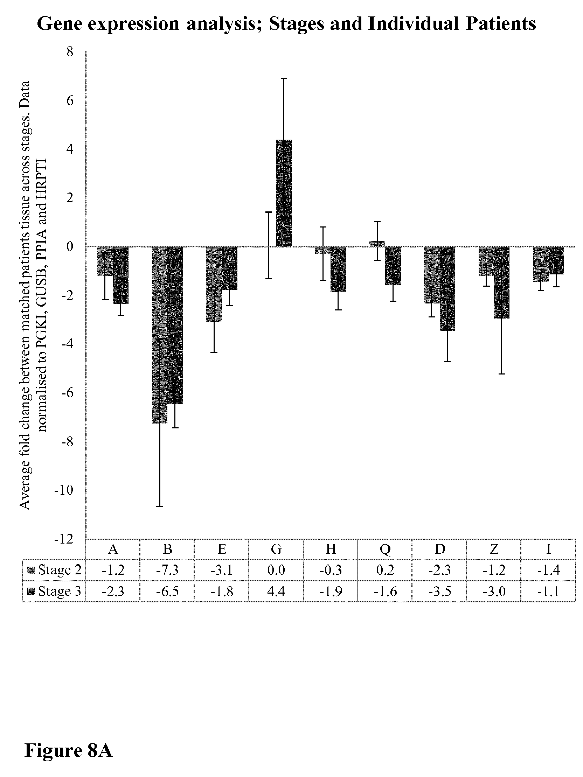

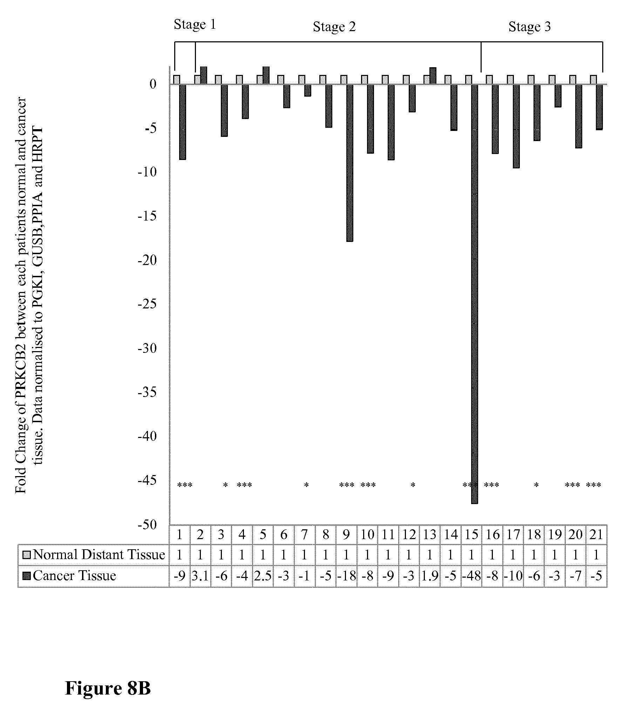

Figure 8. Gene expression analysis. (A) Average fold change of 9 PKC coding genes in cancer tissue of patients with stage 2 (n=14) and stage 3 (n=6) colorectal cancer. Each individual patients fold change was analysed using the REST© software (No statistical significance in expression between stages for any of the PKC coding genes as determined by paired student T-test). (B) Fold change of PRKCB2 in cancer tissue of each patient. Results were obtained by comparing mRNA level of PRKCB2 in individuals normal tissue compared to levels in that individuals cancer tissue (Statistical significance based on Pair Wise Fixed Reallocation Randomisation Test©, *p<0.05 and ***p<0.01).

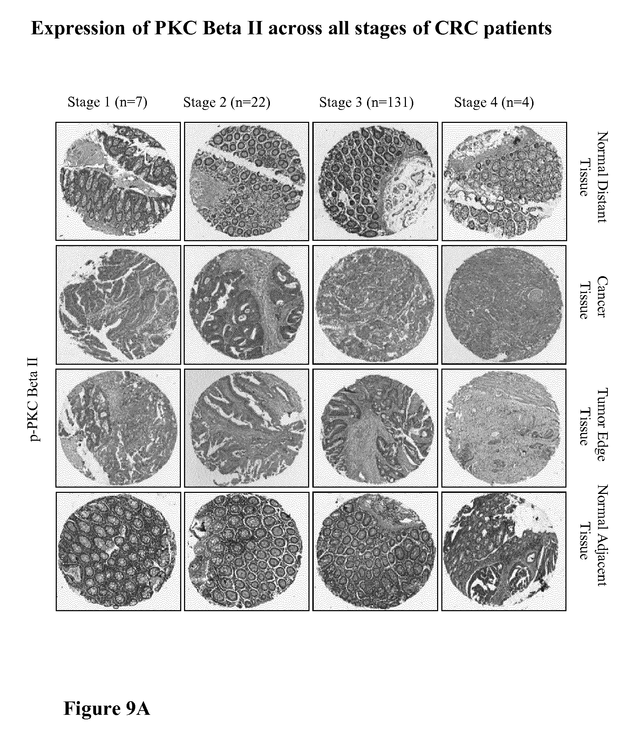

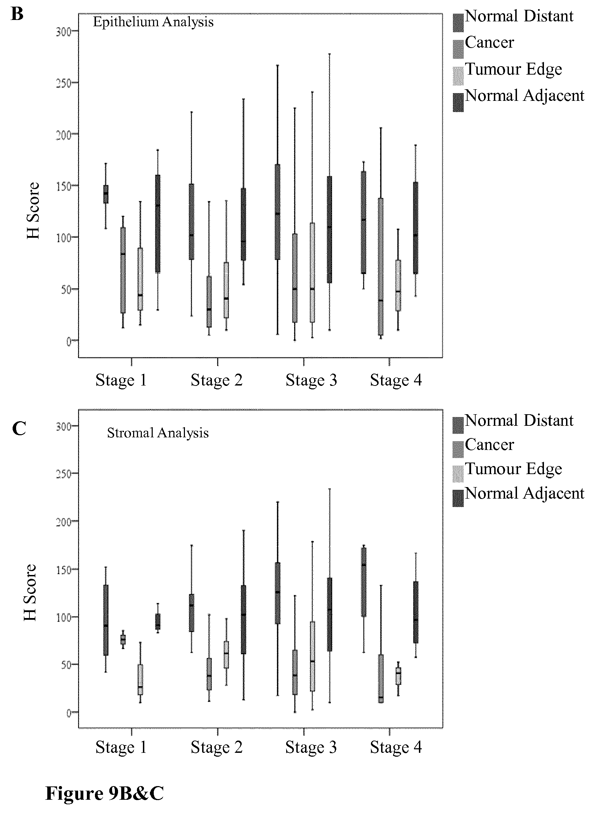

Figure 9. Expression of PKC Beta II across different stages of CRC patients. PKC Beta II expression was assessed by immunohistochemistry in normal distant and cancer tissue samples in stages 1, 2, 3 and 4 of CRC patients. (A) Representative image of PKC Beta II staining for Normal Distant, Cancer tissue, Tumour Edge and Normal Adjacent tissue in stage 1 (n=6, 7,7,3), stage 2 (n=21, 22,12,16), stage 3 (n=120,131,101,101) and stage 4 (n=4, 4,3,4) CRC patients. (B) Box plot representing the difference in PKC Beta II H score in Normal Distant, Cancer, Tumour Edge and Normal Adjacent epithelium tissue across stage 1, stage 2, stage 3 and stage 4 CRC patients (No statistical difference in expression between stages as determined by Welch test and Bonferroni test). (C) Box plot representing the difference in PKC Beta II H score in Normal Distant, Cancer, Tumour Edge and Normal Adjacent stromal tissue across stage 1, stage 2, stage 3 and stage 4 CRC patients (No statistical difference in expression between stages as determined by Welch test and Bonferroni test).

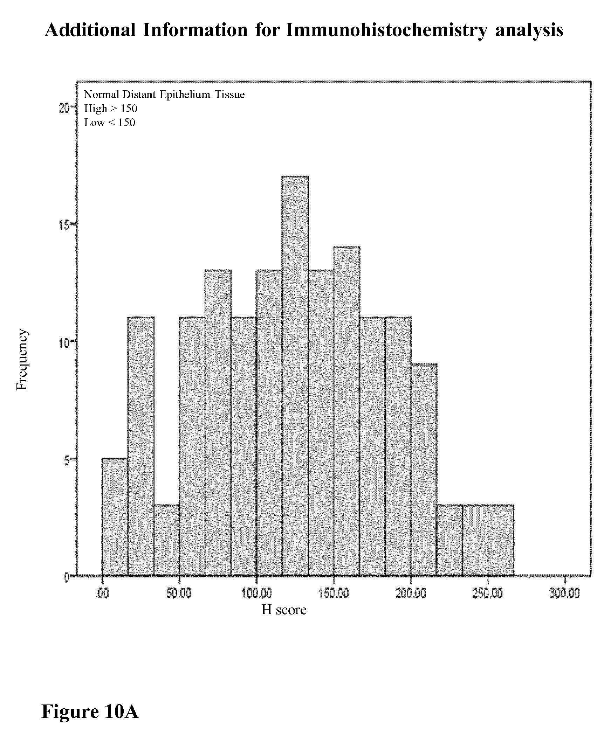

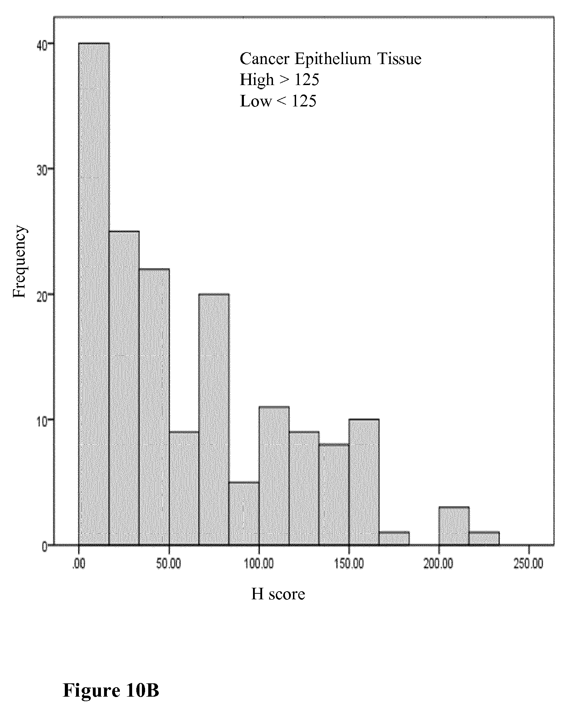

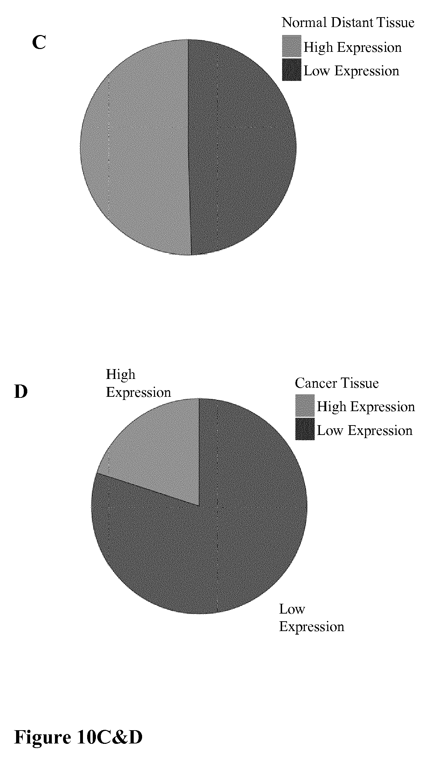

Figure 10. Immunohistochemistry analysis. (A and B) Histograms used to determine the high and low expression values of PKC Beta II in (A) normal distant epithelium tissue and (B) cancer epithelium tissue. (C and D) Pie chart representing the % of cases with high and low expression in the normal distant (C) and cancer tissue (D).

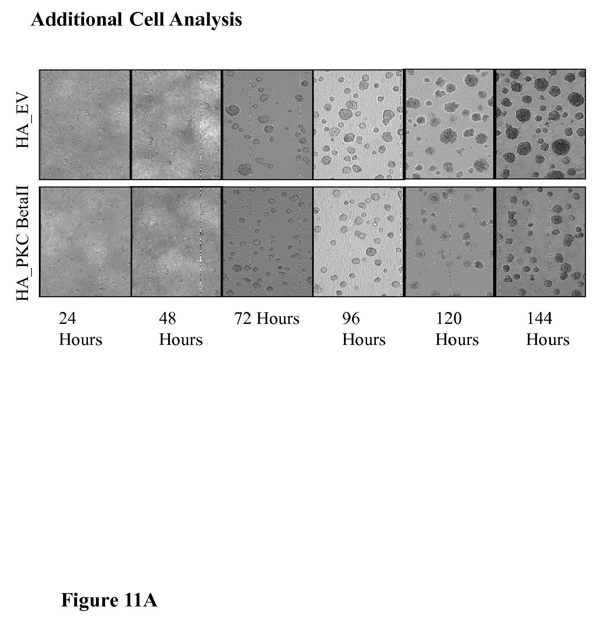

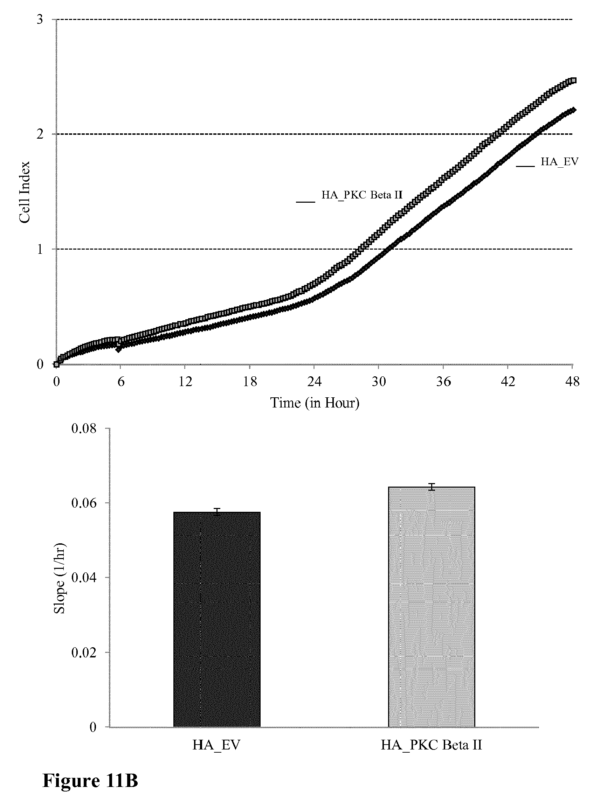

Figure 11. Additional Cell Analysis. (A) Images demonstrating the difference in the size of cells growing in a 3D matrix at 24 hour intervals. (B) Representative graph of cells proliferating over a period of 48 hours analysed in real time on the xCELLigence system. Bar graph represents the rate of proliferation as determined by analysing the slope of the line between the 12 and 48 hour interval (no statistical difference found).

The invention will be more clearly understood from the following description of some embodiments thereof, given by way of example only, with reference to the following figures in which:

### Detailed Description of the Invention

The present inventors examined colorectal cancer patients and found not only that PKC Beta II is a tumour suppressor, but patients with low levels of this isozyme in their normal distant epithelial tissue when compared with levels in their cancer epithelial tissue have significantly decreased disease free survival. Specifically, analysis of gene expression levels of all PKC genes in matched normal and cancer tissue samples from colorectal cancer patients revealed a striking down-regulation of the gene coding PKC Beta in the cancer tissue (n = 21). Tissue microarray analysis revealed a dramatic down-regulation of PKC Beta II protein levels in both the epithelial and stromal diseased tissue (n=166). Of clinical significance, low levels of the protein in the normal tissue of patients is associated with a low (10%) 10 year survival compared with a much higher (60%) survival in patients with relatively high levels of the protein. Consistent with PKC Beta II levels protecting against colorectal cancer, overexpression of PKC Beta II in colorectal cancer cell lines reveals that PKC Beta II reverses transformation in cell based assays. Further to this, activation of PKC Beta II in an IGF-1 dependent manor showed a downregulation of AKT, indicating a role for PKCs in regulating IGF-1 mediated cell survival. Thus, the present inventors have identified that PKC Beta II is a tumour suppressor in colorectal cancer and that low levels of PKC Beta II in a patient's normal distant tissue serve as a predictor for poor survival outcome.

A sample of normal distant tissue may be defined as a sample taken 5-15cm (+/-2cm) from the colorectal cancer tissue in the patient. In certain embodiments, the sample may be taken 8-12 cm away from the colorectal cancer tissue in the patient. In certain embodiments, the sample may be taken 10 cm away from the colorectal cancer tissue in the patient.

The biological sample of colorectal cancer and/or the biological sample of normal distant tissue may comprise RNA coded by the PRKCB gene or fragments thereof. PRKCB is the gene which encodes PKC Beta II. The method may comprise determining PKC Beta II gene expression levels in the samples. This may be carried out using gene expression profiling methods known to persons skilled in the art, for example, a PCR-based method or any other method of nucleic acid amplification.

The biological sample of colorectal cancer and/or the biological sample of normal distant tissue may comprise PKC Beta II protein. The method may comprise determining PKC Beta II protein levels. This may be carried out using methods known to persons skilled in the art, for example, using a kit that comprises a primary specific anti-PKC Beta II antibody, a secondary antibody capable of conjugating with the primary antibody, and other reagents and additives required to determine the expression level of PKC Beta II in samples. Typically the secondary antibody is labelled to allow the antibody to be detected.

PKC Beta II expression and/or activity of PKC Beta II may be upregulated by administering a drug which upregulates PKC Beta II expression and/or activity or by administering PKC Beta II itself, either in protein form or as gene therapy.

### Definitions

As used herein, the term "colorectal cancer" or "CRC" is understood to refer to bowel cancer. This term includes colon and rectal cancer. The "colorectal cancer tissue" may be colon tissue and/or rectal tissue.

The term "normal" when used herein to refer to tissue is understood to mean tissue which is not part of a tumour or cancer tissue.

The term "normal distant tissue" as used herein is understood to refer to non-cancerous tissue which is taken 5-15cm (+/- 2cm) from a patient's colorectal cancer tissue. The term "normal adjacent tissue" as used herein is understood to refer to non-cancerous tissue which is taken 0-5 cm (+/- 2cm) from a patient's colorectal cancer tissue. Typically the measurement refers to the length of tissue, e.g. colon, between a tumour and the normal tissue when the tissue is removed from the patient. Normal distant tissue does not include normal adjacent tissue which is found closer to the tumour than normal distant tissue. Normal distant tissue and normal adjacent tissue include epithelial and stromal tissue.

"Stromal" tissue is defined herein as the part of the tissue that has a structural and connective role and doesn't carry out the specific function of the organ. Cells found in the stroma include fibroblasts and pericytes. Colorectal cancer does not originate in the stromal tissue, but the stroma contributes massively to disease progression.

"Epithelial" tissue is defined herein as tissue which is composed of epithelial cells which carry out the specific function of the organ. Colorectal cancer originates in the epithelial tissue as a result of the acquisition of one or several mutations. The progression of that cancer is influenced by the composition and nature of the stromal tissue.

Typically the terms "subject" and "patient" are used interchangeably herein. The subject is typically a mammal, more typically a human.

The term "percentage difference" as used herein refers to a change in value between a first value (e.g. the PKC Beta II level in normal distant epithelium tissue) and a second value (e.g. the PKC Beta II level in cancer epithelium tissue) divided by an average of the two values (e.g. an average of the two levels) and then multiplied by 100.

### Examples

The inventors compared the expression of all PKC isoforms in colorectal cancer (CRC) to understand whether its function was enhanced or reduced in this cancer. As PKCs are differentially expressed across and within different tissues, their approach was to reduce any variability that may occur between patients by examining the expression of the genes encoding PKCs in patients matched normal and colorectal cancer tissue. Their analysis revealed a dysregulation of many of the isoforms with a striking down regulation of the PKC Beta II isoform in cancer tissue. To further investigate the implication of this in colorectal cancer, they examined the protein expression of PKC Beta II across a large cohort of patients and compared expression of PKC Beta II in normal distant tissue, normal adjacent tissue, cancer tissue and tumour edge tissue. Interestingly, this revealed a dramatic downregulation of the protein in the cancer and tumour edge tissue. Further to this, they found a strong association between low levels of PKC Beta II in normal distant tissue and a reduction in disease free survival time for colorectal cancer patients. To substantiate their findings, they overexpressed PKC Beta II in colorectal cancer cells and used a series of cell based assays including Real-Time cell migration and cell invasion assays to reveal that overexpression of PKC Beta II reverses the transformed phenotype. Upon further investigation, they revealed that both activation and overexpression of PKC Beta II dramatically reduced phosphorylation of AKT and consequently reduced cell survival. All these data combined strongly suggest that PKC Beta II has a tumour suppressor role in colorectal cancer.

### Materials and Methods

### Clinical Samples

Following ethical approval tissue samples measuring approximately 0.5cm in diameter were collected from 21 patients (median age 68 y; range, 45-84; male 13, female 8) undergoing surgery in University Hospital Limerick. One patient had stage 1, 14 patients had stage 2 and 6 patients had stage 3 colorectal cancer. Normal tissue from the 21 patients was also collected approximately 10 cm away from the cancer tissue. Specimens were immediately placed in Allprotect tissue reagent (Qiagen) and stored at -80°C.

A second cohort of tissue samples was collected, with ethical approval, from 197 patients (median age, 71 y; range, 34-94; male 109, female 88) undergoing surgery at St. James Hospital Dublin. Seven patients had stage 1, 24 patients had stage 2, 161 patients had stage 3 and 5 patients had stage 4 colorectal cancer. Samples were collected from the tumour, tumour edge, normal distant tissue and normal adjacent tissue. After surgery, samples were fixed in 10% formalin and embedded in paraffin.

### RNA extraction and cDNA synthesis

Frozen tissue was immersed in liquid nitrogen and ground into powder. Lysis buffer was added to tissue and the sample transferred to tubes using a 21-gauge needle. Total RNA was extracted as per Qiagen RNeasy Mini Kit instructions. RNA was quantified using a Nanodrop Spectrophotometer (Thermo Scientific) and stored at - 80 degrees. RNA purity was evaluated by the ratio of absorbance at 260/280 nm and RNA quality was evaluated through visualization of the 28S:18S ribosomal RNA ratio on a 1% agarose gel (Figure 7A). Total RNA (1 µg) was synthesised into cDNA using Vilo cDNA synthesis kit (Invitrogen) and stored at -20 degrees.

### Real-Time PCR

Real-time PCR was conducted using the ABI 7900 HT instrument (Applied Biosystems) following supplier instructions. Taqman® Gene Expression Assay Kits (Applied Biosystems) were used to analyse the gene expression of the 9 coding PKCs genes. The ratio of stromal tissue to epithelium tissue in normal tissue and cancer tissue samples was analyzed using KRT18 and VIM taqman® gene expression assays in 12 random tissue samples (Figure 7B). A panel of nine housekeeping genes were evaluated using excel Normfinder and the four most stable genes were used for normalisation of each experiment (Figure 7C).

### Immunohistochemistry

Tissue microarrays (TMA) were constructed from formalin fixed paraffin-embedded pre-treatment tumour samples using 1 mm cores, and they are in triplicate for each patient sample. Immunohistochemistry was performed using a Vectastain ABC Kit (Elite), as per the manufacturer's instructions. Slides were deparaffinised, rehydrated and heated-antigen retrieval was performed using Trilogy solution (Cell Marque Corporation). Endogenous peroxidase activity was blocked using hydrogen peroxide (3%) for 30 min and sections were blocked using goat serum and incubated with rabbit anti-human phospho-PKC Beta II (phospho S660) (Abcam, 1:300 dilution) for 1 h at room temperature. Sections were then incubated with secondary antibody for 30 min at room temperature. Diaminobenzidine (Sigma) was used to visualize staining and sections were counter-stained with haematoxylin, dehydrated and mounted. Stained sections were scanned using a scanscope XT digital scanner and ImageScope software (Aperio Technologies). Expression was assessed by scoring positive cell count and intensity in the epithelium and stroma by 2 reviewers who were blinded to the TRG status of the patient. Positivity was assessed using 7 categories (0%, 10%, 25%, 50%, 75%, 90% and 100%). Intensity was assessed using a scale of 0, 1, 2 and 3 which correlated with negative, weak, medium and strong staining, respectively. The % distribution and intensity values were multiplied to give the 'H score' for each tissue core.

### Cell Culture and Stable Transfection

HCT 116 colorectal carcinoma cells were purchased from ATCC (ATCC® CCL-247™ with certificates of analysis) and were maintained in Dulbecco's modified Eagle's medium (DMEM) (Sigma), supplemented with 10% (v/v) fetal calf serum, 10 mM L-Glu, and 5 mg/ml penicillin/streptomycin. HCT116 cells were transfected with pcDNA3/HA-PKC Beta II or empty pcDNA3 vector using LipofectAMINE Plus (Invitrogen). 24 h after transfection HCT116 cells were split into medium containing G418 (1.5 mg/ml) and maintained for 14 days with regular replenishment of medium and drug. Cells were examined for the expression of HA-PKC Beta II by western blotting. Cells overexpressing HA-PKC Beta II were maintained in Dulbecco's modified Eagle's medium supplemented with 1.5 mg/ml G418.

### Colony Formation Assay

Stable cell lines were harvested with trypsin/EDTA, washed with DMEM and counted using a haemocytometer. 500 cells were plated per well of a 6 well plate and incubated at 37°C in 5% CO2 for 10 days. Following this time, the cells were fixed in 96% ethanol for 10 min and subsequently stained with 0.05% crystal violet for 20 min. The wells were washed carefully and allowed to dry. Colonies were counted and recorded and a colony was deemed to be of 50 cells or more in size.

### 3-Dimensional Cell cultures

Individual wells of a 6 well plate were coated with MatrigelTM (BD Biosciences) and placed in an incubator at 37°C for 30 min. Stable cell lines were trypsinized and counted. 50,000 cells/ml were resuspended in DMEM supplemented with 2% MatrigelTM. Cells were placed in MatrigelTM coated wells for 30 min at 37°C, after which DMEM supplemented with 2% MatrigelTM was added to the cultures. Cells were maintained in culture for 6 days in an incubator at 37°C, 5% CO₂ with fresh medium added every 2 days and cultures imaged every 24 hours (Olympus cellSens Dimension 1.12). On day 6, cultures were harvested using EDTA/PBS, fixed with paraformaldehyde (PFA) and stained for confocal analysis (Zeiss LSM 710).

### Scratch Wound Assay

Adhesive wound assay inserts (Ibidi) were used to generate wounds in confluent layers in a 6 well plate. To do this, stable cells were trypsinized and seeded into the plate to form a monolayer. After overnight growth and attachment, the insert was carefully removed and the cells were starved of serum by adding serum free media for 4 hours, after which DMEM was added to the cells and they were imaged immediately (TO) using (Olympus cellSens Dimension 1.12). Cells were maintained in an incubator at 37°C, 5% CO₂ for 24 hours, after which time images of the wound closure were taken as described above. The percentage wound closure was analyzed using Wimasis image analysis.

### Real Time Migration and Invasion analysis

The rate of cell migration and invasion was monitored in real-time with the xCELLigence system CIM-plates. 4 hours prior to the experiment stable cell lines were serum starved. The upper chamber (UC) of the CIM-plates was coated with 1 ug/ul of fibronectin and for invasion experiments an additional 1:40 solution of MatrigelTM (BD Biosciences) was added. 30,000 cells were seeded in each well of the UC in serum free media. DMEM was added to each well of the lower chamber (LC). The CIM-plates was left in an incubator for 1 hour to allow cell attachment. The impedance value of each well was automatically monitored by the xCELLigence system (xCELLigence RTCA DP, ACEA) for duration of 72 hours and expressed as a cell index value (Cl) value.

### Real Time Etoposide Assay

The rate of cell death and proliferation was monitored in real time using the xCELLigence E-plate system. Either 30 000 HCT116 cells overexpressing PKC Beta II or 30 000 HCT116 cells expressing empty vector were placed in each well. The cells were allowed to adhere and proliferate for 16 hours following which time 15µM of etoposide (Sigma) was added to the cells. The impedance value of each well was automatically monitored by the xCELLigence system for 32 hours and the data was normalised at the point the etoposide was added.

### IGF-1 and PMA treatment of cells

Cells were starved of serum for four hours prior to treatment with 50ng of IGF-1 for the indicated times. The PKC inhibitor, Gö6983 (1µM), was added 5 minutes prior to phorbol 12-myristate 13-acetate (PMA) and PMA (100nM) was added 15 minutes prior to IGF-1. Cell lysates were prepared and run on 10-12% gradient gels and then transferred to membranes, which were blocked for 1 h at room temperature in TBS containing 0.05% Tween 20 (TBS-T) and 5% milk (w/v). All primary antibody incubations were overnight at 4°C. Secondary antibody incubations were carried out at room temperature.

### FRET Imaging and Analysts

HCT116 cells were rinsed and imaged in Hanks' balanced salt solution containing 1mM Ca2+. Images were acquired and analysed as previously described (Gallegos LL et al., Journal of Biological Chemistry. 2006; 281(41):30947-30956). For PKC activity measurements cells were co-transfected with CKAR and RFP tagged rat PKC Beta II. For AKT activity measurements cells were transfected with plasma membrane targeted BKAR (Kunkel MT et al., Journal of Biological Chemistry. 2005; 280(7):5581-5587). Cells were starved of serum 4 hours prior to treatment of plates with IGF-1.

### Statistical Analysis

Statistical analysis was performed using SPSS 20 Statistical Package unless stated otherwise. Significance between two groups was determined by the Mann Whitney U Test. Significance between more than two group was determined by the Welch Test and differences between these groups were determined by the Bonferroni Test. Disease Free Survival was analyzed using Kalpain Meir survival curves and the Log Rank Test, significance is reflective of univariate analysis. For matched patient samples differences in gene expression levels was determined for each individual patient using Pair Wise Fixed Reallocation Randomisation Test© as per REST© software. For all statistical analysis differences were considered to be statistically significant at p < 0.05.

### Results

### The genes encoding Protein Kinase C isoforms are dysregulated in colorectal cancer

To investigate the change in PKC expression in colorectal cancer, fresh tissue samples that were excised from both the cancer tissue and normal distant tissue of individual patients were used. Using real time PCR, the inventors compared PKC expression in patients' normal distant tissue to their cancer tissue and established an individual fold change for each patient. After averaging the fold change of all patients for each PKC coding gene, PRKCB, PRKCD and PRKCE demonstrated a down-regulation greater than two while PRKCG was upregulated (Figure 1A, Figure 7). This suggests that there is differential expression of the genes encoding PKC when the cancer tissue and normal tissue of individuals are compared. The most striking dysregulation was the downregulation of the gene coding for the protein PKC Beta II. This dysregulation was not influenced by the progression of the disease as no statistical difference was found between stage 2 and stage 3 patients (Figure 8A). To investigate this further, the inventors examined the relative expression of PRKCB in normal distant tissue and compared it to cancer tissue, normal tissue and benign tissue (Figure 1B, Figure 8B). They found a significant downregulation of PRKCB in the cancer tissue compared to the normal distant tissue (p<0.01), with no difference between the other tissue groups examined.

### PKC Beta II is down-regulated in tumours from CRC patients

The changes observed in the expression of PKC Beta II are noteworthy as there are some conflicting results in the literature. Earlier studies suggested that PKC Beta II expression is upregulated and is an early event in colorectal cancer in mice. This considered the inventors next wanted to determine if this downregulation was observed at a protein level in CRC patients. To do this, they examined the expression of mature PKC Beta II in 197 CRC tissue samples (Table 1), using tissue microarrays and immunohistochemistry. As expected, and in support of the gene analysis results, expression of PKC Beta II was significantly reduced in the cancer epithelium tissue when compared with normal distant epithelium tissue (p<0.01) (Figure 2A, 2B). Similar results were found when comparing cancer stromal tissue with normal distant stromal tissue (p<0.01) (Figure 2A, 2B).

**Table 1. Characteristics of the cohort of colorectal cancer (CRC) patients and summary of the pathology of the tumour microarrays used to analysis the expression of PKC Beta II.**

| | **Stage 1** | **Stage 2** | **Stage 3** | **Stage 4** |
|---|---|---|---|---|
| **N** | 7 | 24 | 161 | 5 |
| **Male/Female** | 5\2 | 7\17 | 96\65 | 1\4 |
| **Age, median (range)** | 69 (45-75) | 65 (45-86) | 72 (34-94) | 67 (53-92) |
| **CRC mortality** | 0 | 8 | 105 | 4 |
| **Lymphvascular Invasion** | 0 | 2 | 136 | 0 |
| **Metastatic** | 0 | 6 | 24 | 5 |

In addition, the inventors examined the expression of mature PKC Beta II in tumour cores representative of normal adjacent and tumour edge tissue. Again, expression was significantly reduced in the tumour edge epithelium tissue (p<0.01) when compared with normal distant epithelium and normal adjacent epithelium tissue, while no significant difference was observed between tumour edge epithelium and cancer epithelium tissue (Figure 2A, 2B). Similar results were also obtained for the stromal tissue (Figure 2A, 2B). Comparable to the inventors' gene expression analysis, progression of the disease did not influence the protein levels of PKC Beta II (Figure 9). To the best of the inventors' knowledge, their data show for the first time that PKC Beta II is down-regulated at both the gene and protein level in CRC tissue.

### Low expression of PKC Beta II in normal distant epithelium tissue is associated with a reduction in disease free survival time

To test whether a low expression of PKC Beta II was associated with a poor prognosis, the inventors compared the survival of patients with low and high expression of PKC Beta II. Low and high expression values were determined using histograms with values less than the median designated low expression and those greater than the median designated high expression (Figure 10A, 10B). An expression value was defined as high where the value was higher than the value against which it was compared and the percentage difference therebetween was more than 10%. An expression value was defined as low where the value was lower than, or similar to, the value against which it was compared. Values were considered similar if the percentage difference therebetween was 10% or less. Remarkably, although fewer patients had low expression of PKC Beta II in their normal distant tissue (Figure 10C, 10D), those patients that did have low expression in their normal distant epithelium tissue showed a significant reduction in their disease free survival time (p<0.01) (Figure 3A). Interestingly, this was only true for the normal distant epithelium tissue as no significant difference was observed between high and low expression in the normal distant stromal tissue (Figure 3B). Moreover, there was no reduction in the disease free survival time associated with low and high expression in either the cancer epithelial tissue (Figure 3C) or the cancer stromal tissue (Figure 3D). This reflects the inventors' findings that the downregulation of PKC Beta II is not progressive across tumour stage (Figure 8A and Figure 9). Consequently, as expected, lower expression in the cancer epithelium is not associated with a reduction in disease free survival.

### Overexpression of PKC Beta II reverses the transformed phenotype of HCT116 cells

The inventors' data indicate that there is a downregulation of PKC Beta II in colorectal cancer. To test whether overexpressing PKC Beta II in colorectal cancer cells would reverse the transformed phenotype, they generated HCT116 cells overexpressing PKC Beta II (Figure 4A) and carried out a number of cell based assays. Using colony formation assays, they observed that cells overexpressing PKC Beta II show a significant reduction in the ability to form colonies (p<0.05) (Figure 4B). Next, they examined whether cells overexpressing PKC Beta II would show altered behaviour in 3-Dimensional culture. Strikingly, 3D cultures overexpressing PKC Beta II showed a 50% reduction in culture size after 144 hours (Figure 4C, Figure 11A). In an attempt to get a more comprehensive view of the phenotype of the 3D cultures, cultures were extracted at 144 hours, fixed, stained and observed using confocal microscopy. Cultures expressing empty vector demonstrated a migratory phenotype, reminiscent of wild type HCT116 cultures. However, in cells overexpressing PKC Beta II, the cultures were more organised and remained small and rounded (Figure 4C).

Next, the inventors tested whether PKC Beta II overexpression would have an influence on the migratory capacity of HCT116 cells. To do this, the inventors carried out a wound migration assay using ibidi® inserts and observed a significant reduction in the ability of cells overexpressing PKC Beta II to migrate into the wound (p<0.01) (Figure 4D). Their observations were due to the decreased migratory behaviour of the overexpressing cells as no difference was observed in the rate of proliferation (Figure 11 B). Following this, the inventors monitored the migratory behaviour of the cells in real time over using the xCELLigence CIM-plates configuration. Serum starved overexpressing cells were placed in the upper chamber of the CIM-plates with 10% FBS acting as a chemoattractant in the lower chamber. Strikingly, cells overexpressing PKC Beta II showed an 80% reduction in the ability to migrate towards the chemoattractant after 72 hours (p<0.01) (Figure 4E). To test if the invasive property of HCT116 cells was altered in the overexpressing cells, a similar assay was set up, with an additional matrigel layer added to the upper chamber of the CIM-plates to stimulate the ECM and to provide a barrier for the cells to invade. As above, cells overexpressing PKC Beta II demonstrated a significant reduction in the rate of invasion towards the chemoattractant (p<0.01) (Figure 4F). All this information considered would indicate that PKC Beta II plays an important role in maintaining the malignant phenotype of HCT116 carcinoma cells as overexpression of PKC Beta II results in the reversal of this phenotype.

### Overexpression and activation of PKC Beta II reduces cell survival and IGF-1 mediated phosphorylation of AKT

All these results combined indicate that PKC Beta II is acting as a tumour suppressor in colorectal cancer. To gain an insight into the mechanistic role PKC Beta II plays, the inventors examined the effect of PKC Beta II on the survival pathway in HCT116 cells using a number of different approaches. Firstly, using the xCELLigence system they observed that etoposide induced an increase in cell death in cells stably overexpressing PKC Beta II when compared to empty vector (p<0.01) (Figure 5A). This result indicates that PKC Beta II plays a role in reducing the survival pathway in colorectal cancer cells. To investigate this further, they examined the effect PKC activation had on the IGF-1 induced phosphorylation of AKT. HCT116 cells were treated with PMA or DMSO for 15 minutes prior to IGF-1 at different time intervals. Interestingly, cells pretreated with PMA showed a dramatic downregulation of IGF-1 induced phosphorylation of AKT (Figure 5B). This observation led the inventors to predict that HCT116 cells stably overexpressing PKC Beta II would show reduced phosphorylated AKT compared to empty vector cells. To examine this, the inventors treated both groups of cells with IGF-1, and observed a marked decrease in pAKT expression in the HCT116 cells stably overexpressing PKC Beta II (Figure 5C). To confirm this decrease in pAKT was induced by PKC activation, they next treated cells with the PKC inhibitor, Gö6983, five minutes prior to PMA treatment. As predicted, they observed that the PMA effect was abolished when cells were pretreated with Gö6983 (Figure 5D, 5E). To distinguish the effect IGF-1 and PMA had on PKC activity, they utilized the FRET-based PKC reporter (CKAR) which specifically uses changes in FRET to quantify PKC phosphorylation activity in real-time and in live cells. The phosphorylation-dependent FRET ratio changes revealed that IGF-1 had no effect on PKC activity and the activity was specifically induced by PMA and inhibited by Gö6983 (Figure 5F). Next, using an AKT-specific FRET reporter (BKAR), the inventors compared the effect of PMA treatment on IGF-1 induced activity of AKT. Strikingly, they observed that PMA treatment showed a dramatic reduction in the phosphorylation activity of AKT compared to untreated HCT116 cells (Figure 5G). The downregulation of PKC Beta II at both a gene and protein level in a large cohort of patients, combined with the reversal of the malignant phenotype of colorectal cancer cells and the dramatic effect it has on reducing the survival pathway in colorectal cancer cells, indicates that PKC Beta II is in fact functioning as a tumour suppressor. Together, these data demonstrate that in tumour cells, loss of PKC Beta II is an early event in cancer progression which leads to increased survival and maintenance of the transformed phenotype.

### Discussion

Using matched normal and cancer tissue from CRC patients, the inventors have shown dysregulation of PKC coding genes in colorectal cancer patients with the most striking difference being a downregulation of PKC Beta II. The inventors' findings also show that PKC Beta II is dramatically down-regulated at the protein level in the cancer tissue of a large cohort of CRC patients and most interestingly, low expression of the protein in normal distant tissue is associated with a reduction in disease free survival. Further to this, overexpression of PKC Beta II radically decreases cell migration and invasion while also reducing cell survival in colorectal cancer cells. These results challenge traditional views that PKC isoforms act as tumour promoters, and instead demonstrate that PKC Beta II acts as a tumour suppressor.

The inventors' comprehensive gene expression analysis of all PKC isoforms revealed that three of the nine PKC coding genes (PRKCB, PRKCD and PRKCE) showed a down-regulation greater than two in the cancer tissue, while only one of the genes (PRKCG) was up-regulated. It is also noteworthy that all other isoforms were trending towards down-regulation in the cancer tissue suggesting that PKCs have a tumour suppressor role in colorectal cancer tissue.

The inventors next looked at PKC Beta II expression at the protein level using a large number of cancer and normal tissue samples obtained from CRC patients (Table 1). They found that there was a striking down-regulation of the mature protein in tissue taken from both the tumour itself and tissue taken from the tumour edge. Interestingly also, this reduction in levels of PKC Beta II was observed in both the epithelial and stromal tissue suggesting that PKC Beta II down-regulation across the entire tumour is an important feature of colorectal cancer. This is valuable information as it is becoming more apparent that the stromal environment is an integral part of cancer initiation and progression.

One of the most intriguing findings of the inventors was the strong association found between low levels of PKC Beta II in normal distant tissue and a reduction in disease free survival time for CRC patients. This may explain why patients receiving PKC inhibitors have reduced disease control rates. It is not surprising that no relationship was found between levels of PKC Beta II in cancer tissue and disease free survival time, as the inventors clearly found that the down-regulation of the protein is not enhanced by disease progression.

Considering the data, what effect might an increase in PKC Beta II levels have on colorectal cancer cells? In agreement with the inventors' proposed hypothesis that PKC Beta II inhibits tumour growth, colorectal cancer cells overexpressing the protein showed a dramatic decrease in the ability to form colonies in plating efficiency assays. The migratory and invasive capacities of these cells, as monitored by Real-Time cell analysis were also severely compromised. Moreover, when the inventors overexpressed PKC Beta II in colorectal cancer cells there was a marked reduction in the size of their 3-dimensional cultures and a dramatic change in the migratory phenotype of the cultures. Taken together, this demonstrates that increasing levels of PKC Beta II in colorectal cancer cells reverses the malignant phenotype of the cells and further points to a tumour suppressor role for PKC Beta II.

What mechanistic role does PKC Beta II exhibit in order to suppress this cancer phenotype? A common advantageous hallmark of cancer cells is upregulated survival signalling and a resistance to cell death. Considering that the loss of PKC Beta II appears to be an important early step in the formation of the transformed phenotype, the inventors anticipated that PKC Beta II may play an important role in inhibiting the survival pathway. Consistent with this, they found that cells overexpressing PKC Beta II showed a reduction in their resistance to cell death induced by the common cytotoxic anti-cancer drug, etoposide. A common mechanism for the promotion of cell survival in cancer is through AKT. In agreement with this, the inventors observed a marked downregulation of IGF-1 mediated phosphorylation of AKT when PKCs were activated in colorectal cancer cells and in cells overexpressing PKC Beta II. Accordingly, this reduction of AKT phosphorylation was reversed when the cells were treated with a PKC inhibitor. They observed that activation of PKC by PMA was independent of IGF-1 and IGF-1 mediated activation of AKT was significantly reduced in the presence of PMA. These data would strongly suggest that PKC Beta II plays an important role in suppressing the survival pathway in cancer cells making the downregulation of PKC Beta II an imperative early step in the establishment and maintenance of the transformed phenotype (Figure 6).

The inventors' patient analysis and in vivo assays provide a strong supporting argument that PKC Beta II functions as a tumour suppressor in the prevention of colorectal cancer. This contrasts with previous work suggesting the up-regulation of PKC Beta II is an early event in colorectal cancer in mice (Gokmen-Polar Y et al., Cancer research. 2001; 61(4):1375-1381). Taken together; the inventors' findings that PKC Beta II is reduced in the stromal and epithelial tissue of the tumours and that low expression of PKC Beta II in normal distant tissue of CRC patients dramatically reduces disease free survival time points strongly towards the idea that a 'global' down-regulation of PKC Beta II is an important driver of the disease. This provides a good explanation as to why clinical trials with PKC inhibitors have been largely unsuccessful and some in fact reduced disease control rates.

The inventors' work strongly highlights that PKC Beta II has an important role to play in the suppression of colorectal cancer. PKC Beta II may in fact be acting to control the activity of phosphatases which subsequently regulate AKT and reduce cell survival (Figure 6). This challenges the current view of PKCs and how PKCs are targeted in the clinic.

## Claims

1. A method for making a prognosis of colorectal cancer in a patient suffering from colorectal cancer, the method comprising steps of:
- determining PKC Beta II levels in a biological sample of colorectal cancer epithelial tissue from the patient;
- determining PKC Beta II levels in a biological sample of normal distant epithelial tissue from the patient; and
- comparing determined levels of PKC Beta II in the cancer and normal distant epithelial tissue samples to make the prognosis.

2. The method as claimed in claim 1 wherein the prognosis is poor where determined levels of PKC Beta II in the normal distant epithelial tissue sample are lower than determined levels of PKC Beta II in the cancer sample or where a percentage difference between determined levels of PKC Beta II in the normal distant epithelial tissue sample and determined levels of PKC Beta II in the cancer sample is 10% or less.

3. The method as claimed in claim 2 wherein a poor prognosis predicts a disease free survival period of less than ten years.

4. The method as claimed in claim 1 wherein the prognosis is good where determined levels of PKC Beta II in the normal distant epithelial tissue sample are higher than determined levels of PKC Beta II in the cancer sample and a percentage difference therebetween is more than 10%.

5. The method as claimed in claim 4 wherein a good prognosis predicts a disease free survival period of more than ten years.

6. The method as claimed in any one of claims 1-5 wherein the sample comprising normal distant epithelial tissue is a sample taken 5-15cm (+/- 2cm) from the colorectal cancer tissue in the patient.

7. The method as claimed in claim 6 wherein the sample comprising normal distant epithelial tissue is a sample taken 8-12cm from the colorectal cancer tissue in the patient.

8. The method as claimed in any one of claims 1-7 wherein the method comprises determining PKC Beta II gene expression levels.

9. The method as claimed in any one of claims 1-7 wherein the method comprises determining PKC Beta II protein levels.

10. A method for determining a suitable treatment for a patient suffering from colorectal cancer, the method comprising steps of:
- determining PKC Beta II levels in a biological sample of colorectal cancer tissue from the patient;
- determining PKC Beta II levels in a biological sample of normal tissue from the patient; and
- comparing determined levels of PKC Beta II in the cancer and normal tissue samples to determine a suitable treatment.

11. The method as claimed in claim 10 wherein if determined levels of PKC Beta II in the normal tissue sample are higher than determined levels of PKC Beta II in the colorectal cancer sample and a percentage difference therebetween is more than 10%, a suitable treatment comprises upregulating expression and/or activity of PKC Beta II.

12. The method as claimed in claim 10 or 11 wherein the sample of colorectal cancer tissue is epithelial tissue, stromal tissue or a mixture thereof and the sample of normal tissue is epithelial tissue, stromal tissue or a mixture thereof.

13. The method as claimed in any one of claims 10-12 wherein the method comprises determining PKC Beta II gene expression levels.

14. The method as claimed in any one of claims 10-12 wherein the method comprises determining PKC Beta II protein levels.

15. PKC Beta II or a drug which upregulates expression and/or activity of PKC Beta II for use in treatment of colorectal cancer in a subject in need thereof.
